# FASCICULE DE BREVET EUROPEEN

(11) **EP 3 772 346 B1**
(45) Date de publication et mention de la délivrance du brevet: **11.05.2022**
(21) Numéro de dépôt: 20189331.0
(22) Date de dépôt: 04.08.2020
(51) Int. Cl.: A61B 17/68, A61B 17/88

(54) **FIXATEUR DE VOLET CRÂNIEN**
SCHÄDELDECKENFIXIERER
CRANIAL FLAP FIXATIVE

(30) Priorité: 06.08.2019 FR 1909023
(43) Date de publication de la demande: 10.02.2021
(73) Titulaire: Vitalys Surgical, 35500 Vitré (FR)
(72) Inventeur: COLLERAIS, Pierre-Yves, 35500 VITRE (FR)
(74) Mandataire: Vidon Brevets & Stratégie

(56) Documents cités:
- WO-A1-2004/089231
- DE-A1-102008 050 870
- DE-U1-202007 007 880
- US-A- 5 449 361
- US-A1- 2018 296 247

## Description

### Domaine technique

La présente technique se rapporte au domaine des dispositifs chirurgicaux implantables.

Plus précisément, la présente technique concerne les dispositifs chirurgicaux classiquement utilisés par les neurochirurgiens lors des opérations/interventions de craniotomie/craniectomie dans le but de remettre en place et fixer un volet crânien préalablement retiré afin de dégager une voie d'abord et de traiter une pathologie.

De tels dispositifs sont habituellement dénommés fixateurs de volets crâniens et sont destinés à positionner et maintenir en place le volet crânien au sein de l'orifice crânien, postérieurement à l'opération/intervention chirurgicale traitant la pathologie.

### Art antérieur

Les fixateurs de volet crânien sont largement utilisés pour positionner et maintenir en place au sein d'un orifice crânien un volet crânien préalablement retiré lors d'une craniotomie. Leur utilisation est généralement fiable et relativement aisée pour le chirurgien. En général, trois ou quatre fixateurs sont utilisés sur un même volet crânien, pour coopérer avec respectivement trois ou quatre perforations préalablement effectuées grâce à un perforateur crânien

Chaque fixateur de volet crânien comporte généralement une capsule inférieure destinée à être placée manuellement par le chirurgien sous la périphérie de l'orifice crânien, entre la dure-mère et la surface inférieure de l'os crânien, ci-après dénommé crâne. Lorsque la capsule inférieure de chaque fixateur est positionnée et que le volet crânien est remis en place dans l'orifice crânien, une capsule supérieure peut être amenée en contact contre les surfaces supérieures de l'os crânien et du volet crânien. Les capsules inférieure et supérieure de chaque fixateur sont ensuite comprimées l'une contre l'autre afin de maintenir en place le volet crânien dans l'orifice crânien.

Une fois les capsules positionnées, la tige rigide doit être sectionnée au ras de la capsule supérieure à l'aide d'une pince coupante.

De tels fixateurs sont notamment commercialisées par la société Aesculap AG sous la dénomination commerciale CranioFix^{®}2.

Un inconvénient de cette technique réside dans le fait que le titane utilisé pour fabriquer le fixateur produit des artéfacts voire une zone d'ombre lors d'examens par rayonnements (IRM, scanner, radiographie, etc...) qui sont amenés à être effectués en post-opératoire.

Un autre inconvénient de cette technique réside dans le fait que les différents instruments spécifiques utilisés doivent être stérilisés avant chaque utilisation.

Une deuxième technique commercialisée sous la dénomination « Cranial Loop » par la société NEOS Surgery S.L propose de solidariser les capsules inférieure et supérieure au moyen de deux tiges souples faites dans un matériau polymère par exemple. Ces fixateurs sont pourvus d'une poignée à usage unique en forme de boucle qui permet de déplacer la capsule supérieure le long des tiges souples en direction de la capsule inférieure. Les tiges souples sont ensuite sectionnées au ras de la capsule supérieure en pliant celle-ci deux ou trois fois.

Un inconvénient de cette technique réside dans le fait que, lors de son utilisation, le chirurgien doit utiliser ses deux mains pour la bonne application du fixateur. On entend par application du fixateur la compression des capsules inférieure et supérieure contre le volet crânien. Plus précisément, l'outil se présente sous la forme d'une boucle de préhension dont chacune des extrémités est reliée à l'une des tiges souples. Pour appliquer le fixateur, le chirurgien doit donc maintenir la capsule supérieure d'une main et tirer sur la boucle de préhension d'une autre main afin de déplacer la capsule supérieure vers la capsule inférieure. Les deux mains du chirurgien sont donc requises pour la mise en œuvre du fixateur, ce qui n'est pas satisfaisant.

Notons que pour chacune de ces techniques, l'excédent de tige doit être coupé. Pour la seconde technique, il est alors nécessaire de couper les extrémités des deux tiges souples, ce qui nécessite un temps d'opération supplémentaire de la part du chirurgien, et cela pour chaque fixateur.

Une autre technique commercialisée sous la dénomination « Aesculap CranioFix^{®} absorbable» par la société Aesculap AG consiste à fabriquer les capsules inférieure et supérieure dans un matériau résorbable tel que le polyester de sorte que le fixateur se résorbe de lui-même après un certain nombre de semaines. Selon cette technique, les capsules sont reliées entre elles par une pluralité de fils de suture.

Cette technique consistant à mettre en œuvre des fixateurs résorbables est particulièrement adaptée en pédiatrie afin de ne pas gêner ou compromettre la croissance du crâne de l'enfant. Néanmoins, les fixateurs résorbables ne sont généralement pas préconisés chez l'adulte. De plus, la solidarisation des capsules inférieure et supérieure par suture nécessite une nouvelle fois les deux mains du chirurgien. Enfin, on notera que ces capsules inférieure et supérieure doivent présenter une épaisseur importante résultant du caractère résorbable du matériau qui les compose.

On observe de plus qu'en pratique il est souvent difficile pour le neurochirurgien de positionner correctement la capsule inférieure contre la paroi intérieure du crâne. En effet, cette paroi peut présenter des surépaisseurs anatomiques ou des creux résultant de la craniectomie. La capsule inférieure étant imparfaitement positionnée, la capsule supérieure l'est également du fait de la rigidité du concept. Les capsules inférieure et supérieure peuvent ne sont ainsi pa parallèles entre elles et peuvent se trouver en porte-à-faux. Ce mauvais positionnement peut conduire à des défauts de cicatrisation et donc à des complications pouvant avoir des effets dommageables pour le patient.

Pour tenter de résoudre ce problème, on connaît de DE102008050870A1 un fixateur dont la tige est reliée à la capsule inférieure de sorte à permettre une inclinaison de la tige par rapport à la capsule inférieure et ainsi d'améliorer le positionnement de celle-ci. Toutefois, cette solution n'est pas complètement satisfaisante car elle met en œuvre des capsules en titane ou autre matériau rigide. Cette rigidité des capsules empêche d'optimiser le positionnement de la capsule inférieure, et corollairement celui de la capsule supérieure, pour adapter parfaitement le fixateur à l'anatomie de la paroi intérieure et de la paroi extérieure du crâne.

Les capsules en titane ou en tout autre matériau rigide présentent de plus l'inconvénient de faire saillie de la surface du crâne. Elles sont en effet fabriquées par emboutissage sous presse et présentent de ce fait une certaine épaisseur liée à leur forme. Après repositionnement du scalp, les capsules supérieures forment des bosses visibles et détectables au toucher qui constituent une source d'inconfort physique et psychologique pour le patient et peuvent même contribuer à créer un certain préjudice esthétique. Les capsules inférieures font quant à elle saillie de la surface intérieure du crâne ce qui peut provoquer des problèmes de tolérance.

Il existe ainsi un besoin pour un fixateur de volet crânien permettant le positionnement optimisé de ses capsules pour une adaptation parfaite à l'anatomie des parois intérieures et extérieurs du crâne.

Il existe également un besoin pour un fixateur dont la capsule supérieure, une fois recouverte du scalp, ne forme pas de bosse et est indétectable visuellement ou au toucher.

Il existe aussi un besoin pour un tel fixateur qui soit plus simple à manipuler que les fixateurs de l'art antérieur.

### Résumé de l'invention

La présente technique permet de résoudre au moins certains des inconvénients soulevés par l'art antérieur. La présente technique se rapporte en effet, à un fixateur de volet crânien comprenant une tige rigide, une capsule inférieure prévue à une extrémité distale de ladite tige rigide, une capsule supérieure montée mobile le long de ladite tige rigide, et une poignée reliée à l'extrémité proximale de ladite tige rigide permettant de faire coulisser ladite capsule supérieure vers ladite capsule inférieure ladite extrémité distale de ladite tige rigide étant dotée d'un élément de rotule articulé dans une gorge et ladite capsule inférieure comprenant une gorge de réception dudit élément de rotule.

Ladite capsule inférieure et ladite capsule supérieure présentent des formes convexes.

Selon la technique proposée, celles-ci sont réalisées dans un matériau leur permettant de se déformer pour s'adapter aux formes du crâne et du volet crânien, de façon telle qu'après application, les capsules inférieure et supérieure sont plaquées et calées contre des surfaces intérieure et extérieure du crâne et du volet crânien, les capsules et le crâne s'inscrivant alors dans un même profil. Elles épousent ainsi parfaitement la forme du crâne, sans dépasser de celui-ci (« profil 0 »).

Selon l'invention, la tige rigide est articulée dans la gorge de la capsule inférieure de sorte à permettre une inclinaison de la tige rigide par rapport à la capsule inférieure. Ainsi la capsule inférieure peut s'adapter à la surface intérieure du crâne et à la surface intérieure du volet crânien. Cette polyaxialité de la tige rigide permet de positionner la capsule inférieure au mieux par rapport à la surface intérieure du crâne et du volet crânien. De plus, la forme et le matériau des capsules autorisent une déformation de celles-ci qui permet aux capsules, lors de leur application, d'être plaquées et calées respectivement contre les surfaces intérieure et extérieure du crâne et du volet crânien, de façon à pouvoir s'adapter complètement à ces surfaces. Ainsi, après positionnement de ces capsules, la capsule inférieure ne forme aucune bosse et, une fois recouverte du scalp ne forme aucune protubérance détectable visuellement ou au toucher, pour un meilleur confort du patient. La capsule inférieure se confondant avec la surface intérieure du crâne elle interagit moins avec les tissus sous-jacents à celui-ci ce qui permet d'améliorer sa tolérance et de diminuer les risques de complication à ce niveau.

Ainsi, le positionnement de la capsule inférieure et de la capsule supérieure peut être amélioré et s'avère donc plus efficace que celui obtenu grâce aux dispositifs de l'art antérieur. Le fixateur selon l'invention est donc plus fiable que ceux de l'art antérieur en ce sens que son utilisation est susceptible de diminuer les risques liés à un positionnement imparfait des capsules et contribue de plus à une meilleure tolérance de celles-ci

La technique proposée fournie une solution facile à l'emploi ne nécessitant aucun ancillaire pour laquelle aucun instrument accompagnant la pose de l'implant n'est requis.

Selon l'invention, ladite poignée présente un élément creux cylindrique pourvu de deux ailettes de préhension et un piston coulissant dans ledit élément creux cylindrique.

De cette manière, la poignée peut être manipulée d'une seule main. Le chirurgien peut tenir la poignée avec deux doigts (le majeur et l'index par exemple) positionnés sous les ailettes et concomitamment appuyer sur le piston avec le pouce de la même main. La manipulation d'une telle poignée s'apparente ainsi à celle d'une seringue.

Dans un mode de réalisation particulier, ladite capsule inférieure et ladite capsule supérieure du fixateur présentent chacune une pluralité de pétales s'étendant sur leur périphérie. De tels pétales contribuent, avec le matériau constituant les capsules, à la souplesse de celles-ci permettant leur déformation lors de leur application. Une telle conformation en pétales permet d'obtenir une déformation des capsules variable radialement d'un pétale à l'autre et ainsi de parfaire l'adaptation de ces capsules à la conformation anatomique des surfaces intérieure et extérieure du crâne.

Selon un aspect particulier, les capsules et la tige rigide sont e fabriquées dans un matériau biocompatible, biostable et ne produisant pas d'artéfact aux examens par rayonnements. Il est ainsi radio-transparent et résistant.

Ainsi, les capsules et la tige rigide sont fabriquées dans un matériau parfaitement adapté pour être implanté dans le corps humain sans produire aucun effet néfaste chez le patient.

Selon un aspect particulièrement intéressant, le capsules et la tige sont fabriquée en matériau Peek Optima^{®} (marque déposée).

Un tel matériau a pour avantage d'être biocompatible, biostable et de ne pas produire d'artéfact aux examens par rayonnements.

Selon une variante de l'invention, ledit élément de rotule et ladite gorge sont configurés pour autoriser une inclinaison, et donc une polyaxialité, de la tige rigide par rapport à un plan longitudinal de la capsule inférieure d'un angle α pouvant varier entre 0 et 20°.

Un tel angle d'inclinaison permet de faciliter l'insertion de la capsule inférieure sous la boîte crânienne lors de la mise en place du fixateur. Cet angle permet également de positionner de manière optimale la capsule inférieure lors de l'application/la compression du fixateur.

Selon un aspect particulier, ladite tige rigide est une crémaillère et ladite capsule supérieure présente au moins au moins deux, préférentiellement quatre, mâchoires, chacune desdites mâchoires étant pourvue d'au moins un ergot, préférentiellement deux, conçus pour coopérer avec des crans de ladite crémaillère.

Ainsi, la capsule supérieure peut se déplacer uniquement en direction de la capsule inférieure. En effet, l'au moins un ergot (on pourra prévoir notamment deux ergots) et la crémaillère sont configurés pour permettre un déplacement de la capsule supérieure vers la capsule inférieure et pour empêcher un déplacement de la capsule supérieure dans une direction opposée à la capsule inférieure. Cette solution technique relativement simple permet de verrouiller de manière automatique la position de la capsule supérieure sur la tige rigide.

Selon un aspect particulier, ladite capsule inférieure est fixée à ladite extrémité distale de ladite tige rigide.

De cette manière, seule la capsule supérieure peut se déplacer le long de la tige rigide. Le mécanisme est donc relativement fiable et simple à mettre en œuvre.

Selon un autre aspect particulier, ledit élément creux cylindrique comprend des moyens de solidarisation de ladite extrémité proximale de ladite tige rigide.

De cette manière, la poignée, sur laquelle est fixée ladite tige rigide lors de l'application du fixateur, peut être aisément désolidarisée de la tige rigide après la compression des capsules contre le volet crânien et le crâne.

Selon encore un autre aspect particulier, lesdits moyens de solidarisation présentent une lumière taraudée coopérant avec une portion filetée de ladite extrémité proximale de ladite tige rigide.

Une fois que la poignée a été actionnée pour rapprocher et compresser les capsules l'une contre l'autre, la poignée peut être retirée facilement. Pour ce faire, il suffit de la remonter le long de la tige pour dégager de celle-ci. La tige rigide peut alors être sectionnée au ras de la capsule.

Selon un autre aspect, ladite poignée est une poignée à usage unique. Elle est ainsi avantageusement réalisée en un matériau plastique rigide. Elle pourra ainsi par exemple être fabrique en acrylonitrile butadiène styrène (ABS).

De cette manière, tout risque sanitaire est limité. En effet, le fixateur est fourni en un seul élément dans lequel la tige rigide est directement fixée à la poignée. L'ensemble est stocké dans un emballage stérilisé de sorte à ce que le chirurgien puisse l'utiliser de manière sûre.

### Liste des Figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
[Fig 1] illustre en perspective un fixateur selon la technique proposée dans sa position initiale ;
[Fig 2] illustre une vue en coupe transversale du fixateur de la figure 1 lors de sa mise en œuvre contre un volet crânien ;
[Fig 3] illustre une vue en coupe transversale d'un fixateur selon la technique proposée dans une position intermédiaire, lors de sa mise en œuvre contre un volet crânien ;
[Fig 4] illustre une vue en coupe transversale d'un fixateur selon la technique proposée dans une position finale, lors de sa mise en œuvre contre un volet crânien ;
[Fig 5] illustre une vue en perspective inférieure de la capsule supérieure du fixateur représenté aux figures 1 à 4.

### Description détaillée de l'invention

### Structure du fixateur

Le principe général de la technique proposée repose sur la combinaison de la mise en œuvre de capsules déformables sur leurs périphéries extérieures et d'une liaison de type rotule à polyaxialité au niveau de la jonction entre la capsule inférieure et la tige du fixateur. La technique proposée repose aussi sur la mise en œuvre d'une poignée permettant d'utiliser le fixateur d'une seule main.

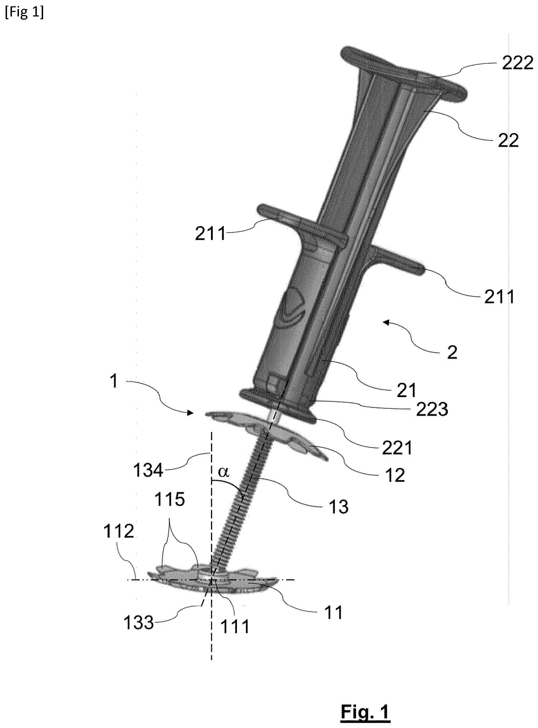

Les **figures 1 à 3** illustrent un fixateur 1 selon l'invention.

Comme illustré sur la **figure 1****,** le fixateur 1 comprend une capsule inférieure 11 se présentant sensiblement sous la forme d'un disque. Une capsule supérieure 12, également en forme de disque, est montée coulissante le long d'une tige rigide 13 dont l'extrémité distale est fixée à la capsule inférieure 11. Le fixateur comprend par ailleurs une poignée 2.

Les capsules inférieure 11 et supérieure 12 sont légèrement convexes avant application du fixateur 1 et présentent une pluralité de pétales 115, 122 s'étendant sur la périphérie de la capsule.

La forme convexe des capsules 11 et 12 et le matériau dans lequel elles sont réalisées leur permettent de légèrement se déformer afin que les pétales 115, 122 s'adaptent aux formes du crâne et du volet crânien C. Ainsi, après application, les capsules 11, 12 sont plaquées/calées de manière optimale contre les surfaces intérieure et extérieure du crâne et du volet crânien C. Ainsi, les capsules et le crâne s'inscrivent dans le même profil.

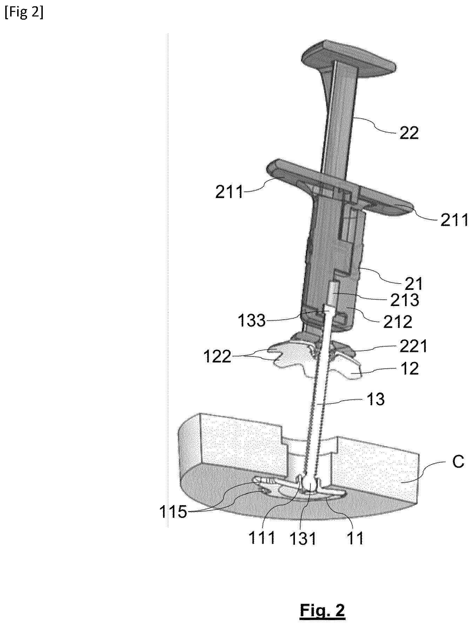

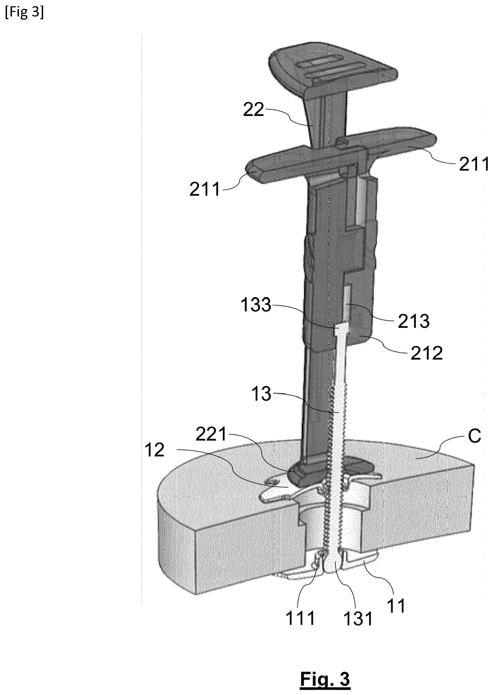

Comme illustré sur les **figures 2** **et** **3** notamment, l'extrémité distale de la tige rigide 13 est fixée à la capsule inférieure 11. Plus précisément, cette extrémité de la tige rigide 13 présente un élément de rotule 131. La capsule inférieure 11 présente, sensiblement en son centre, une rainure, ou gorge, 111 conformée pour recevoir et maintenir l'élément de rotule 131 de la tige rigide 13. La gorge 111 présente une forme sensiblement circulaire. L'élément de rotule 131 et la gorge 111 permettent à la tige rigide 13 de s'incliner par rapport au plan longitudinal 112 de la capsule inférieure 11. Plus précisément, la tige rigide 13 peut s'incliner d'un angle α compris entre 0 et 20° autour d'un axe 134 s'étendant perpendiculairement au plan longitudinal 112 de la capsule inférieure 11, l'axe 134 passant de préférence par le centre de la capsule inférieure 11. L'élément de rotule 131 et la gorge 111 assurent une polyaxialité de la tige rigide 13 par rapport à la capsule inférieure 11.

On notera par ailleurs que ni l'élément de rotule 131, ni la gorge 111 ne font saillie de la surface extérieure de la capsule inférieure. Au contraire, cette gorge 111 est prévue entièrement du coté intérieure concave de la capsule inférieure, et la rotule accueillie par cette gorge est donc elle-même positionnée de ce côté concave.

La tige rigide 13 se présente plus particulièrement sous la forme d'une crémaillère. En d'autres mots, la surface extérieure de la tige rigide 13 présente une pluralité de crans 132 réguliers. Par exemple, les crans 132 présentent une forme sensiblement triangulaire.

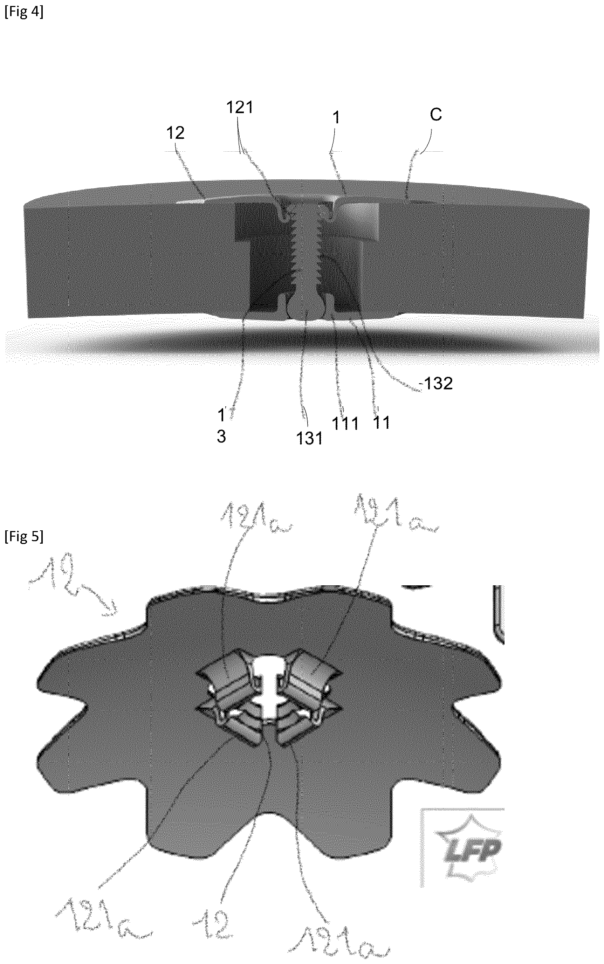

En référence à la figure 5, la capsule supérieure 12 comprend, en son centre, une ouverture/lumière permettant le passage de la tige rigide 13. Plus particulièrement, l'ouverture présente quatre mâchoires 121a pourvue chacune de deux ergots 121 de coopération avec les crans 132 de la crémaillère.

On notera que ni les mâchoires 121a ni les ergots 121 de celles-ci ne font saillie de la surface extérieure de la capsule supérieure. Au contraire, ces mâchoires 121a et ces ergots 121 sont prévus entièrement du coté intérieure concave de la capsule supérieure.

Dans cet exemple, quatre mâchoires 121a pourvue chacune de deux dents s'étendant sur l'ensemble de la périphérie de l'ouverture pour le passage de la tige sont prévus. Dans une variante, un nombre différent de mâchoire(s) ou d'ergot(s) pourra ^ être mis en œuvre sur la périphérie de cette ouverture.

Les crans 132 de la crémaillère et l'ergot 121 sont configurés pour permettre un déplacement/coulissement de la capsule supérieure 12 le long de la tige rigide 13 en direction de la capsule inférieure 11. En outre, les crans 132 de la crémaillère et l'ergot 121 sont configurés pour empêcher que la capsule supérieure 12 ne s'éloigne de la capsule inférieure 11. En d'autres termes, les crans 132 de la crémaillère et l'ergot 121 empêchent un déplacement de la capsule supérieure 12 dans une direction opposée/inverse à la capsule inférieure 11.

Lors de l'application des capsules, il est donc simplement nécessaire de déplacer/rapprocher la capsule supérieure 12 vers la capsule inférieure 11. En d'autres termes, aucune action/manipulation supplémentaire n'est nécessaire pour verrouiller la position de la capsule supérieure 12 sur la tige rigide 13. En effet, le déplacement de la capsule supérieure 12 dans une direction opposée à la capsule inférieure 11 n'est pas autorisé par le mécanisme de crémaillère et d'ergot 121.

Selon la technique proposée, le fixateur 1 comprend une poignée 2, ou applicateur, à usage unique. Cette poignée permet au chirurgien d'appliquer les capsules d'une seule main.

La poignée 2 comprend un corps, ou élément, creux cylindrique 21 présentant deux ailettes 211 de préhension qui permettent au chirurgien de tenir la poignée 2 grâce à deux de ces doigts (le majeur et l'index, par exemple). Les ailettes 211 permettent également de tirer la tige rigide 13, et donc la capsule inférieure 11, afin de correctement plaquer la capsule inférieure 11 contre la surface inférieure du crâne et du volet crânien C.

La poignée 2 comprend en outre un piston, ou poussoir, coulissant 22 traversant le corps creux 21. Le piston 22 est apte à coulisser dans le corps creux 21 lorsqu'une force est appliquée par le chirurgien sur son extrémité supérieure 222 prenant sensiblement la forme d'un bouton poussoir.

L'extrémité inférieure 223 du piston 22 présente un élément d'appui 221, en forme de rondelle fine, destiné à venir en contact avec la capsule supérieure 12. Ainsi, lorsque le piston 22 est déplacé dans le corps creux cylindrique 21, l'élément d'appui 221 vient en contact avec la capsule supérieure 12 et pousse/déplace cette dernière en direction de la capsule inférieure 11, comme illustré sur les **figures 2** **et** **3****.** On notera que l'élément d'appui 221 en forme de rondelle est d'un diamètre plus large que le diamètre des perforations effectués lors de la craniectomie. En pratique, ce diamètre sera avantageusement de 17 mm de diamètre alors que des perforations dans le crâne sont, chez l'adulte d'au plus 14 mm de diamètre. Ainsi, lors de l'utilisation du fixateur selon l'invention par le neurochirurgien qui utilisera la poignée pour comprimer les capsules, cet élément d'appui en forme de rondelle viendra, en compression maximale, s'asseoir largement sur le dessus de la capsule supérieure sans risquer d'effondrer celle-ci en son centre. Ainsi, le neurochirurgien pourra évaluer le bon déroulement de cette étape non seulement par un contrôle visuel mais aussi par un contrôle auditif et tactile.

Lors de l'utilisation, le neurochirurgien tient la poignée 2 par deux doigts via les ailettes 211 et pousse sur le piston 22 via le bouton poussoir 222, avec son pouce par exemple. Ainsi, il rapproche le bouton poussoir 22 des ailettes 211, ce qui rapproche les capsules inférieure 11 et supérieure 12 l'une de l'autre, jusqu'à compression des capsules 11 et 12 contre le crâne et le volet crânien C.

Le corps creux cylindrique 21 comprend en outre des moyens de solidarisation 212 de l'extrémité proximale de la tige rigide. Ces moyens permettent de maintenir la tige rigide 13 lorsque la capsule supérieure 12 est déplacée en direction de la capsule inférieure 11 lors de l'application du fixateur 1.

Dans cet exemple, l'extrémité proximale de la tige rigide 13 présente une portion filetée 133 destinée à coopérer avec une lumière taraudée 213 ménagée dans les moyens de solidarisation 212 du corps creux cylindrique 21. Le fixateur 1 et la poignée 2 peuvent donc être solidarisés/désolidarisés par vissage/dévissage.

On comprend bien évidemment que d'autres solutions peuvent être envisagées pour solidariser la tige rigide 13 à la poignée 2. Par exemple, on pourrait prévoir un système de clipsage/clippage ou un système à goupille.

Le fixateur, γ inclus sa poignée, est avantageusement à usage unique afin de s'affranchir de tout risque sanitaire lors de son utilisation.

Le fixateur 1 est de préférence fabriqué dans un matériau biocompatible et biostable de sorte à diminuer les risques de rejet par le corps du patient et ainsi les complications à la suite de l'intervention.

De plus, le matériau utilisé pour fabriquer le fixateur est de préférence un matériau ne produisant pas d'artéfact aux examens par rayonnements (IRM, scanner ou radiographie, par exemple).

De préférence, le fixateur est fabriqué en matériau PEEK OPTIMA^{®} (marque déposé). Ce matériau particulier présente l'avantage d'être biocompatible, biostable et de ne pas produire d'artéfact aux examens par rayonnements.

### Utilisation du fixateur

Le fixateur 1 de la technique proposée est destiné à fixer un volet crânien C, visible sur les **figures 2 à 4****,** au sein d'un orifice crânien (non illustré) lors d'une craniotomie. Plus précisément, plusieurs fixateurs sont mis en œuvre pour fixer le volet crânien C. Leur nombre est fonction des dimensions du volet crânien C, notamment.

Ainsi, pour fixer un volet crânien C, il est nécessaire de disposer plusieurs fixateur 1 sur la périphérie de l'orifice crânien en fonction du nombre de trépanation. Plus précisément, pour chaque fixateur 1, il est nécessaire que la capsule inférieure 11 soit insérée/disposée à l'intérieur du crâne, entre la dure-mère et la surface intérieure du crâne. Le volet crânien C peut ensuite être remis en place au sein de l'orifice crânien.

L'élément de rotule 131 permet, à ce stade, une inclinaison de la tige rigide 13 de sorte à optimiser le positionnement de la capsule inférieure 11 contre la surface intérieure du crâne et la surface intérieure du volet crânien C comme illustré sur la figure 2. Les surfaces intérieures sont les surfaces à l'intérieur du crâne, c'est-à-dire orientées vers le cerveau du patient.

Lorsque le volet crânien C est correctement mis en place dans l'orifice crânien, la capsule supérieure 12 doit être mise en contact et compressée contre la surface extérieure du crâne et la surface extérieure du volet crânien C. Pour ce faire, il suffit au chirurgien de saisir la poignée 2 du fixateur 1 et de pousser sur le piston 22. Plus précisément, le chirurgien peut effectuer cette opération d'une seule main. En effet, il lui suffit de maintenir la poignée 2 avec deux doigts (l'index et le majeur, par exemple) via les ailettes de préhension 211 et d'appuyer/pousser sur le piston 2, avec son pouce par exemple.

L'application d'une force sur le piston 22 entraine un déplacement du piston 22 en direction du volet crânien C. Ce déplacement du piston 22 entraine ainsi un déplacement de la capsule supérieure 12 le long de la tige rigide 13 en direction de la capsule inférieure 11. En d'autres termes, la poignée 2 peut être utilisée de façon relativement similaire à une seringue, ce qui s'avère être simple et efficace pour le chirurgien. La poignée 2 de la technique proposée permet ainsi de déplacer la capsule supérieure 12 en ne nécessitant qu'une seule main du chirurgien, ce qui facilite l'application du fixateur 1. Cette application est encore facilitée par le fait que le mode de réalisation du fixateur ici présenté est très léger et ne pèse que cinq grammes environ.

Le déplacement du piston 22, et donc de la capsule supérieure 12, est effectué jusqu'à ce que la capsule supérieure 12 soit compressée/comprimée contre la surface supérieure du volet crânien C, comme illustré sur la **figure 3****.** Au cours du déplacement de la capsule supérieure 12 sur la tige rigide 13, l'ergot 121 franchit un à un les crans 132 de la crémaillère en direction de la capsule inférieure 11. Un déplacement de la capsule supérieure 12 dans la direction inverse est empêché grâce à la forme particulière des crans 132 et de l'ergot 121, comme décrit précédemment.

Lors de la compression des capsules inférieure 11 et supérieure 12 contre le volet crânien C et le crâne, la tige rigide 13 retrouve/reprend une orientation perpendiculaire par rapport au plan longitudinal 112 de la capsule inférieure 11. L'élément de rotule 131 ne permet donc une inclinaison de la tige rigide 13 qu'au cours de la fixation du fixateur 1. Lorsque le fixateur 1 est appliqué, c'est-à-dire dans sa position intermédiaire (**figure 3**) ou finale (**figure 4**), la tige rigide 13 n'est plus inclinée par rapport à la capsule inférieure 11. Les capsules inférieure 11 et supérieure 12 s'étendent donc sensiblement parallèlement l'une par rapport à l'autre.

Lors de la compression des capsules inférieure 11 et supérieure 12 contre le volet crânien C et le crâne, les capsules se déforment légèrement pour s'adapter complètement au surfaces extérieures et intérieures de ceux-ci. Les capsules, le volet crânien et le crâne s'inscrivent alors dans un même profil.

L'élément d'appui 221 du piston 22 ayant une taille supérieure à celle des perforations faites lors de la craniectomie, la capsule supérieure ne peut être écrasée et compressée au-delà de la surface du crâne lorsque le neurochirurgien actionne le piston, ce qui sécurise son intervention.Le fait que cet élément d'appui 221 dépasse de part et d'autre de la perforation du crâne est donc très sécurisant pour le neurochirurgien.

Notamment, la capsule supérieure 12, une fois recouverte du scalp du patient, ne forme pas de bosse détectable visuellement ou au toucher. La capsule inférieure ne fait quant à elle quasiment pas saillie vers l'intérieur du crâne ce qui ménage ainsi les tissus sous-jacents tels que la dure-mère.

Une fois les capsules inférieure 11 et supérieure 12 comprimées contre le crâne et le volet crânien C, la poignée 2 peut être aisément retirée. La tige ainsi dégagée peut ensuite être sectionnée à l'aide d'une pince coupante au ras de la capsule supérieure.

Après que le fixateur 1 ait été désolidarisé de la poignée 2, la portion excédentaire de la tige rigide 13 s'étendant au-delà de la capsule supérieure 12 peut être coupée, comme illustré sur la (**figure 4**). On entend par portion excédentaire, la portion de la tige rigide 13 s'étendant entre la capsule supérieure 12 et l'extrémité proximale présentant la portion filetée 133 de la tige rigide 13. En d'autres termes, la tige rigide 13 est coupée au ras de la capsule supérieure 12.

## Revendications

1. Fixateur (1) de volet crânien (C) sur un crâne présentant au moins une perforation comprenant une tige rigide (13), une capsule inférieure (11) prévue à une extrémité distale de ladite tige rigide (13), une capsule supérieure (12) montée mobile le long de ladite tige rigide (13), et une poignée (2) reliée à l'extrémité proximale de ladite tige rigide (13) permettant de faire coulisser ladite capsule supérieure (12) vers ladite capsule inférieure (11), ladite extrémité distale de ladite tige rigide (13) étant dotée d'un élément de rotule (131) et ladite capsule inférieure (11) comprenant une gorge (111) de réception dudit élément de rotule (131),
ladite capsule inférieure (11) et ladite capsule supérieure (12) présentant des formes convexes,
**caractérisé en ce que** ladite capsule inférieure (11) et ladite capsule supérieure (12) sont réalisées dans un matériau leur permettant de se déformer pour s'adapter aux formes du crâne et du volet crânien, de façon telle qu'après application, les capsules inférieure (11) et supérieure (12) sont plaquées et calées contre des surfaces intérieure et extérieure du crâne et du volet crânien (C), les capsules et le crâne s'inscrivant alors dans un même profil,
et **en ce que** ladite poignée (2) présente un élément creux cylindrique (21) pourvu de deux ailettes (211) de préhension et un piston (22) coulissant dans ledit élément creux cylindrique (21), ledit piston (22) ayant une extrémité supérieure (222) formant un bouton poussoir configuré pour s'adapter au pouce d'un chirurgien et une extrémité inférieure (223) présentant un élément d'appui (221) en forme de rondelle destiné à venir en contact avec la capsule supérieure (12).

2. Fixateur (1) selon la revendication 1 **caractérisé en ce que** ladite capsule inférieure (11) et ladite capsule supérieure (12) présentent chacune une pluralité de pétales (115, 122) s'étendant sur leur périphérie.

3. Fixateur (1) selon l'une des revendications 1 ou 2, **caractérisé en ce que** ledit fixateur (1) est fabriqué dans un matériau biocompatible, biostable et ne produisant pas d'artéfact aux examens par rayonnements.

4. Fixateur (1) selon l'une des revendications 1 à 3, **caractérisé en ce que** ledit élément de rotule (131) et ladite gorge (111) sont configurés pour autoriser une inclinaison de la tige rigide (13) par rapport à un plan longitudinal (112) de la capsule inférieure (11) d'un angle (α) pouvant varier entre 0 et 20°.

5. Fixateur (1) selon la revendication 1 à 4, **caractérisé en ce que** ladite tige rigide (13) est une crémaillère et **en ce que** ladite capsule supérieure (12) présente au moins deux mâchoires, chacune desdites mâchoires étant pourvue d'au moins un ergot (121) conçu pour coopérer avec des crans (132) de ladite crémaillère.

6. Fixateur (1) selon l'une des revendications 1 à 5, **caractérisé en ce que** ladite capsule inférieure (11) est fixée à ladite extrémité distale de ladite tige rigide (13).

7. Fixateur (1) selon l'une des revendications 1 à 6, **caractérisé en ce que** ledit élément creux cylindrique (21) comprend des moyens de solidarisation (212) de ladite extrémité proximale de ladite tige rigide (13).

8. Fixateur (1) selon la revendication 7, **caractérisé en ce que** lesdits moyens de solidarisation (212) présentent une lumière taraudée (213) coopérant avec une portion filetée (133) de ladite extrémité proximale de ladite tige rigide (13).

9. Fixateur (1) selon l'une des revendications 1 à 8, **caractérisé en ce qu'**il est à usage unique.

## Patentansprüche

1. Fixateur (1) einer Schädelklappe (C) auf einem Schädel, der mindestens eine Perforation aufweist, umfassend einen starren Stab (13), eine untere Kapsel (11), die an einem distalen Ende des starren Stabs (13) vorgesehen ist, eine obere Kapsel (12), die beweglich entlang des starren Stabs (13) befestigt ist, und einen Griff (2), der mit dem proximalen Ende des starren Stabs (13) verbunden ist, der ermöglicht, dass die obere Kapsel (12) in Richtung der unteren Kapsel (11) geschoben wird, wobei das distale Ende des starren Stabs (13) mit einem Kugelgelenk-Element (131) ausgestattet ist und wobei die untere Kapsel (11) eine Kehle (111) zur Aufnahme des Kugelgelenk-Elements (131) aufweist,
wobei die untere Kapsel (11) und die obere Kapsel (12) konvexe Formen aufweisen,
**dadurch gekennzeichnet, dass** die untere Kapsel (11) und die obere Kapsel (12) aus einem Material erstellt sind, das ihnen ermöglicht, sich zu verformen, um sich an die Formen des Schädels und der Schädelklappe derart anzupassen, dass die untere Kapsel (11) und die obere Kapsel (12) nach dem Anbringen gegen Innen- und Außenflächen des Schädels und der Schädelklappe (C) gedrückt und verkeilt werden, wobei die Kapseln und der Schädel dann in ein und demselben Profil registriert sind,
und dadurch, dass der Griff (2) ein hohles zylindrisches Element (21) aufweist, das mit zwei Flügeln (211) zum Greifen und einem Kolben (22), der in dem hohlen zylindrischen Element (21) gleitet, versehen ist, wobei der Kolben (22) ein oberes Ende (222), das einen Druckknopf bildet, der konfiguriert ist, um sich an den Daumen eines Chirurgen anzupassen, und ein unteres Ende (223) aufweist, das ein Auflageelement (221) in Form einer Scheibe aufweist, das dazu bestimmt ist, mit der oberen Kapsel (12) in Kontakt zu kommen.

2. Fixateur (1) nach Anspruch 1, **dadurch gekennzeichnet, dass** die untere Kapsel (11) und die obere Kapsel (12) jeweils mehrere Blätter (115, 122) aufweisen, die sich um ihren Umfang erstrecken.

3. Fixateur (1) nach einem der Ansprüche 1 oder 2, **dadurch gekennzeichnet, dass** der Fixateur (1) aus einem biokompatiblen, biostabilen Material hergestellt ist, das bei Strahlenuntersuchungen keine Artefakte erzeugt.

4. Fixateur (1) nach einem der Ansprüche 1 bis 3, **dadurch gekennzeichnet, dass** das Kugelgelenk-Element (131) und die Kehle (111) dazu eingerichtet sind, eine Neigung des starren Stabs (13) relativ zu einer Längsebene (112) der unteren Kapsel (11) um einen Winkel (α) zu ermöglichen, der zwischen 0 und 20° variieren kann.

5. Fixateur (1) nach Anspruch 1 bis 4, **dadurch gekennzeichnet, dass** der starre Stab (13) eine Zahnstange ist und dass die obere Kapsel (12) mindestens zwei Backen aufweist, wobei jede der Backen mit mindestens einem Sporn (121) versehen ist, der dazu ausgebildet ist, mit den Kerbungen (132) der Zahnstange zusammenzuwirken.

6. Fixateur (1) nach einem der Ansprüche 1 bis 5, **dadurch gekennzeichnet, dass** die untere Kapsel (11) an dem distalen Ende des starren Stabs (13) befestigt ist.

7. Fixateur (1) nach einem der Ansprüche 1 bis 6, **dadurch gekennzeichnet, dass** das hohle zylindrische Element (21) Mittel (212) zum Befestigen des proximalen Endes des starren Stabs (13) aufweist.

8. Fixateur (1) nach Anspruch 7, **dadurch gekennzeichnet, dass** die Befestigungsmittel (212) eine Innengewindeöffnung (213) aufweisen, die mit einem Gewindeabschnitt (133) des proximalen Endes des starren Stabs (13) zusammenwirkt.

9. Fixateur (1) nach einem der Ansprüche 1 bis 8, **dadurch gekennzeichnet, dass** er für den einmaligen Gebrauch bestimmt ist.

## Claims

1. Fixator (1) of a cranial flap (C) to a skull having at least one perforation comprising a rigid rod (13), a lower disk (11) placed at a distal extremity of said rigid rod (13), an upper disk (12) mounted so as to be mobile along said rigid rod (13), and a grip (2) connected to the proximal extremity of said rigid rod (13) enabling said upper disk (12) to be made to slide towards said lower disk (11), said distal extremity of said rigid rod (13) being provided with a ball-joint element (131) and said lower disk (11) comprising a recess (111) for receiving said ball-joint element (131)
**characterized in that** said lower disk (11) and said upper disk (12) have convex shapes and are made out of a material enabling them to get deformed so as to adapt to the shapes of the skull and the cranial flap in such a way that, after application, the lower (11) and upper (12) disks are placed flat against and secured to the internal and external surfaces of the skull and of the cranial flap (C), the disks and the skull then being contained within a same profile,
and **in that** said grip (2) has a hollow cylindrical element (21) provided with two grasping fins (211) and a piston (22) sliding in said hollow cylindrical element (21), said piston (22) having an upper extremity (222) forming a push-button configured to adapt to the thumb of a surgeon and a lower extremity (223) having a supporting element (221) in the shape of a washer intended to come into contact with the upper disk (12) and having a diameter wider than the diameter of said perforation.

2. Fixator (1) according to claim 1 **characterized in that** said lower disk (11) and said upper disk (12) each have a plurality of petals (115, 122) extending on their periphery.

3. Fixator (1) according to any one of the claims 1 or 2, **characterized in that** said fixator (1) is manufactured out of a biocompatible and biostable material that produces no artifact under examinations by radiation.

4. Fixator (1) according to any one of the claims 1 to 3, **characterized in that** said ball-joint element (131) and said recess (111) are configured to allowing a tilting of the rigid rod (13) relative to a longitudinal plane (112) of the lower disk (11) by an angle (α) that can vary between 0° and 20°.

5. Fixator (1) according to claims 1 to 4, **characterized in that** said rigid rod (13) is a rack device and **in that** said upper disk (12) has at least two jaws, each of said jaws being provided with at least one pin (121) designed to cooperate with the notches (132) of said rack device.

6. Fixator (1) according to any one of the claims 1 to 5, **characterized in that** said lower disk (11) is fixed to said distal extremity of said rigid rod (13).

7. Fixator (1) according to any one of the claims 1 to 6, **characterized in that** said hollow cylindrical element (21) comprises means (212) for fixedly attaching said proximal extremity of said rigid rod (13).

8. Fixator (1) according to claim 7, **characterized in that** said means (212) for fixedly attaching have a tapped hole (213) cooperating with a threaded portion (133) of said proximal extremity of said rigid rod (13).

9. Fixator (1) according to any one of the claims 1 to 8, **characterized in that** it is a one-time-use fixator.
